(19) Europäisches Patentamt European Patent Office Office européen des brevets

(11) **EP 4 505 199 B1**

(12) **EUROPEAN PATENT SPECIFICATION**

(45) Date of publication and mention of the grant of the patent:
**03.06.2026   Bulletin 2026/23**

(21) Application number: **23718319.9**

(22) Date of filing: **05.04.2023**

(51) International Patent Classification (IPC):
*G01R 33/00* (2006.01)    *G01R 33/028* (2006.01)
*G01R 33/16* (2006.01)    *A61B 34/20* (2016.01)
*A61B 5/06* (2006.01)    *A61B 90/00* (2016.01)

(52) Cooperative Patent Classification (CPC):
**G01R 33/0017; A61B 5/065; A61B 90/39;**
A61B 2034/2051; A61B 2090/3954; G01R 33/028;
G01R 33/16

(86) International application number:
**PCT/GB2023/050909**

(87) International publication number:
**WO 2023/194727 (12.10.2023 Gazette 2023/41)**

(54) **IMPROVEMENTS IN OR RELATING TO SUSCEPTIBILITY PROBES FOR DETECTING SURGICAL MARKERS**

VERBESSERUNGEN AN ODER IM ZUSAMMENHANG MIT SUSZEPTIBILITÄTSSONDEN ZUM NACHWEIS CHIRURGISCHER MARKER

AMÉLIORATIONS APPORTÉES OU SE RAPPORTANT À DES SONDES DE SUSCEPTIBILITÉ POUR DÉTECTER DES MARQUEURS CHIRURGICAUX

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority:   **05.04.2022   GB 202204999**

(43) Date of publication of application:
**12.02.2025   Bulletin 2025/07**

(73) Proprietor: **Endomagnetics LTD**
**Cambridge CB4 0WN (GB)**

(72) Inventor: **HATTERSLEY, Simon**
**Cambridge Cambridgeshire CB4 OWN (GB)**

(74) Representative: **Abel & Imray LLP**
**Westpoint Building**
**James Street West**
**Bath BA1 2DA (GB)**

(56) References cited:
**WO-A1-2011/067576     WO-A1-2014/140566**
**US-A1- 2012 229 130     US-A1- 2015 338 376**

- **WAANDERS S ET AL: "A handheld SPIO-based sentinel lymph node mapping device using differential magnetometry", PHYSICS IN MEDICINE AND BIOLOGY, INSTITUTE OF PHYSICS PUBLISHING, BRISTOL GB, vol. 61, no. 22, 26 October 2016 (2016-10-26), pages 8120 - 8134, XP020310510, ISSN: 0031-9155, [retrieved on 20161026], DOI: 10.1088/0031-9155/61/22/8120**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

<u>Technical field</u>

The present disclosure relates to a probe for surgical use, for sensing a magnetic marker, and a detection system comprising such a probe.

<u>Background</u>

In the field of susceptibility probes for the detection of magnetic markers, it is known to use a combination of a drive coil and a sense coil to determine the proximity of a magnetic marker to the probe. An AC current may be supplied to a drive coil, thereby generating a magnetic drive field. The magnetic drive field induces a response from the magnetic marker, which, in turn, induces a sense voltage in the sense coil. The sense voltage induced in the sense coil may be processed by a signal processor and a signal indicative of the marker position may be output to a user. Therefore by measuring and interpreting the sense voltage from the sense coil, the proximity of the marker to the probe may be determined.

In known probes, a single drive coil may be positioned juxtaposed a sense coil that is positioned close to a tip of the probe. A relatively long drive coil may be used to generate a relatively strong magnetic drive field, as this improves the sensitivity of the probe, allowing a marker to be detected at a greater distance from the probe. However, the magnetic field generated by a long drive coil will show significant magnetic flux variation. If a long drive coil moves axially (i.e. parallel to its long axis) relative to a magnetic marker, the marker will be subject to varying magnetic flux and reversals in the magnetic flux direction. This leads to variation in the sense voltage induced in the sense coil from a marker. The cubic root of the sense voltage (typically measured in microvolts, $\mu V$) may be used to map variation sense voltage around a probe owing to the large range in sense voltage values that are typically recorded. If the cubic root of the sense voltage is mapped in two dimensions, significant 'side lobes' protruding in a transverse direction (i.e. perpendicular to a longitudinal axis of the drive coil) may be observed. In three dimensions, these side lobes extend circumferentially around the probe. These side lobes result in phase reversals, or phase flips, in the sense voltage measured from a marker, as a probe moves axially relative to the marker alongside the drive coil. These phase flips can interfere with the signal that is output to a user, and can make it challenging for a probe user to determine the position of a marker relative to the probe.

The size of side lobes in the sense voltage, and the extent to which they extend away from the drive coil will be dependent upon the magnitude of the drive field, and the dimensions of the drive coil. When a long drive coil is used, the side lobes in the sense voltage may extend beyond, or outside of, the probe housing. Therefore when the probe moves axially relative to a marker that is outside of the probe housing, phase flips in the sense voltage will occur. This can be avoided by increasing the diameter of the probe housing, such that the side lobes in the sense voltage do not extend beyond the probe housing. In this case, if the probe moves axially relative to a marker that is outside of the probe housing, phase flips will not be observed. The sense voltage may therefore be easier to interpret. However, large diameter probes are often undesirable for use in surgical procedures as they increase the size of surgical incision that is required when a probe is being used.

The extent to which side lobes extend away from the drive coil can be reduced by using a drive coil of a shorter length. However, using a shorter drive coil typically reduces the magnitude of magnetic drive field than can be generated without increasing the drive current and producing excessive thermal effects. Using a smaller magnetic drive field will reduce the sensitivity of the probe.

There remains a need for a probe that does not give rise to phase flips in the sense voltage as the probe moves axially relative to a marker. It is desirable for a probe to have a small diameter, thereby allowing for use in a variety of surgical applications, whilst retaining a high sensitivity, so that a marker can be detected at relatively large distances from the probe.

WO2011/067576 A1 discloses a system and method for locating magnetic material. In one embodiment the system includes a magnetic probe; a power module in electrical communication with the magnetic probe to supply current to the magnetic probe; a sense module in electrical communication with the magnetic probe to receive signals from the magnetic probe; and a processing module (computer) in electrical communication with the power module and the sense module. The processing module (computer) generates a waveform that controls the supply of current from the power module and receives a signal from the sense module that indicates the presence of magnetic material. The magnetic probe is constructed from a material having a coefficient of thermal expansion of substantially 10-6/°C or less and a Young's modulus of substantially 50 GPa or greater. In one embodiment magnetic nanoparticles collect in the lymph nodes. In one embodiment the particles have a mean hydrodynamic diameter of between 5-200 nm.

WO2014/140566 A1 discloses a probe for detecting magnetic particles. In one embodiment, the probe includes; a cylindrical probe core having a first end and a second end, the cylindrical probe core defining two channels for containing coils of wire, one of the channels being adjacent the first end of the cylindrical probe core; two sense coils, one each of the sense coils being located in a respective one of the channels; and two drive coils, one each of the drive coils being co-located with the respective sense coil in a respective one of the channels.

US 2012/229130 A1 discloses apparatus for determining magnetic properties of materials. The apparatus comprises a portable probe, an equipment trolley holding cryogenics and electronics and connecting cables. The probe comprises a drive coil and a correction coil, the drive coil being disposed symmetrically with respect to an inner second-order gradiometer sensor coil. Electrical connectors in the form of 2-metre long Belden (1192A) microphone cables are used to connect the apparatus on the equipment trolley to the drive coil, the correction coil and the sensor coil. The drive coil is driven so as to generate a sinusoidally varying magnetic field. The electronics comprise a flux-locked loop, a SQUID controller, a data acquisition module, which captures and processes the signals and computer. A liquid-nitrogen dewar is supported on the equipment trolley and houses a sensitive SQUID detector and a transfer coil made from copper. Possible applications of the apparatus include an intra-operative tool for sentinel lymph node detection in the treatment of breast cancer, and a non-destructive evaluation tool for detecting voids and defects in aluminium and applications in the aeronautics industry.

WAANDERS S ET AL: "A handheld SPIO-based sentinel lymph node mapping device using differential magnetometry", PHYSICS IN MEDICINE AND BIOLOGY, INSTITUTE OF PHYSICS PUBLISHING, BRISTOL GB, vol. 61, no. 22, 26 October 2016 (2016-10-26), pages 8120-8134, XP020310510, ISSN: 0031-9155, DOI: 10.1088/0031-9155/61/22/8120 describes the use of superparamagnetic iron oxide nanoparticles that have unique, nonlinear magnetic properties as an alternative to gamma-radiation centred approaches and other novel magnetic techniques that are used in sentinel lymph node biopsy. Field amplitudes are limited to 5 mT, which enables handheld operation without additional cooling. Improved mass sensitivity can be obtained without the need for external re-balancing of the probe to negate any influences from the human body. Additionally, the approach can be used to suppress artefacts resulting from the presence of metallic instruments.

Summary of Invention

According to a first and a second aspect, the present invention provides a probe for surgical use, for sensing a magnetic marker, as defined in respective appended independent claims 1 and 2. The probe comprises a first set of coils, the first set of coils comprising a first coil of a first coil type disposed between a first pair of coils of a second coil type that are connected in series. The first coil type may be either a sense coil or a drive coil, and the second coil type may be respectively either a drive coil or a sense coil. Electromagnetically, the two possible arrangements are mutually reciprocal, such that the sense and drive coils may be interchanged and the result will be the same electromagnetically. Preferably however, the first coil type is a sense coil and the second coil type is a drive coil. This may allow a drive coil to be positioned at a distal end of the probe, as disclosed hereinbelow, to maximise a magnetic driving field in the vicinity of the magnetic marker. Further, drive coils typically use a larger gauge of wire than sense coils, to carry more current without excessive heating. Thus, the provision of two drive coils allows more space and potentially more turns to be added. Accordingly, the first set of coils preferably comprises a first sense coil disposed between a first pair of drive coils that are connected in series.

The probe further comprises a balancing element, wherein the balancing element is axially separated from the first set of coils along a length of the probe.

The first set of coils and the balancing element may be suitably configured such that as the probe moves in an axial direction along its length relative to a magnetic marker, the marker induces a sense voltage that shows a single phase change. The balancing element may generate a sense voltage that wholly or partially offsets a sense voltage induced in the sense coil or coils of the first set of coils by the drive coils or coil respectively of the first set of coils. For example, the balancing element may generate a sense voltage that wholly or partially offsets a sense voltage induced in the first sense coil by the first pair of drive coils. This means that the net sense voltage can be wholly, or mostly attributed to voltage induced from a magnetic marker.

According to the first aspect of the present invention, the balancing element comprises a second set of coils comprising a second coil disposed between a second pair of coils. The second coil is of the same coil type as the first coil, and each of the second pair of coils is of the same coil type as each of the first pair of coils.

According to the second aspect of the present invention, the balancing element comprises a second set of coils comprising a second coil that is arranged axially proximate the first set of coils and a third coil that is arranged axially remote from the first set of coils, the arrangement being such that the second coil interposes between the first set of coils and the third coil. The second coil is of the same coil type as the first coil, and the third coil is of the same coil type as each of the first pair of coils.

Suitably, all the coils of one of the first and second types may be connected in series and wound in the same direction. The coils of the other of the second and first types may be connected in anti-series or wound in mutually opposite directions.

Preferably, all the drive coils of the first and second sets of coils are connected in series and the sense coils of the first and second sets are connected in anti-series, especially where each set comprises a single sense coil. However, in some implementations, especially where the each set comprises a single drive coil, all of the sense coils of the first and second sets may be connected in series and the drive coils of the first and second sets connected in anti-series.

Thus, the drive coils of the first and second sets of coils may be connected in series. The sense coil or coils of the second set of coils may be coupled in anti-series with the sense coil or coils of the first set of coils. Alternatively, the sense coil or coils of the second set of coils may be coiled in an opposite direction to the sense coil or coils of the first set of coils.

According to a third aspect, the present invention provides a detection system for locating a magnetic marker, according to appended claim 16.

The system comprises a probe for surgical use. The probe may include any of the features outlined above. The system comprises a magnetic field generator arranged to drive an alternating magnetic field through the drive coil or coils of the first set of coils and the balancing element; preferably through a first pair of drive coils and the balancing element. The system comprises at least one detector arranged to receive a signal indicative of a sense voltage.

It will be appreciated that features described in relation to one aspect of the present disclosure may be incorporated into other aspects of the present disclosure. For example, the system of the disclosure may incorporate features described with reference to the apparatus of the disclosure and vice versa.

## Description of the Figures

Embodiments of the present invention will now be described by way of example only with reference to the accompanying schematic drawings in which:

Fig. 1(a) is a plot showing the cubic root of the sense voltage over 2D space for a probe comprising a single long drive coil and a sense coil;

Fig. 1(b) is a plot showing the side sense response of the probe of Fig. 1(a) for a marker moving axially relative to the probe, plotted for different transverse distances from the axial centre of the probe;

Fig. 2(a) is a plot showing the cubic root of the sense voltage over 2D space for a probe comprising a single short drive coil and a sense coil;

Fig. 2(b) is a plot showing the side sense response for a marker moving axially relative to the probe of Fig. 2(a), plotted for different transverse distances from the axial centre of the probe;

Fig. 3(a) is a plot showing the cubic root of the sense voltage over 2D space for a probe according to an embodiment of the present disclosure, comprising a sense coil sandwiched between a pair of drive coils;

Fig. 3(b) is a schematic diagram showing cubic root of the sense voltage in the region between two adjacent side lobes for the probe of Fig. 3(a);

Fig. 3(c) is a plot showing the side sense response for a marker moving axially relative to the probe of Fig. 3(a), plotted for different transverse distances from the axial centre of the probe;

Fig. 4(a) is a schematic longitudinal section taken through a probe according to an embodiment of the present disclosure, comprising two sets of coils, each set comprising a sense coil sandwiched between a pair of drive coils;

Fig. 4(b) is a plot showing the sensitivity of the probe of Fig. 4(a) as a function of distance from the probe tip;

Fig. 4(c) is a plot showing the sensing range as a function of probe former diameter;

Fig. 4(d) is a plot showing the side sense response as a function of axial position along the probe for a marker moving along the probe of Fig. 4(a); and

Fig. 5 is a schematic longitudinal section through a probe according to an embodiment of the present disclosure, comprising two sets of coils; the first set comprising a sense coil sandwiched between a pair of drive coils and the second set comprising a drive coil and a sense coil.

## Detailed Description

According to a first and a second aspect the present disclosure provides a probe for sensing a magnetic marker. The probe comprises a first set of coils, the first set of coils comprising a first coil of a first coil type disposed between a first pair of coils of a second coil type that are connected in series. The first coil type is either a sense coil or a drive coil. The second coil type is respectively either a drive coil or a sense coil. Preferably, as mentioned above, the first set of coils comprises a first sense coil disposed between a first pair of drive coils that are connected in series.

The probe further comprises a balancing element, wherein the balancing element is axially separated from the first set of coils along a length of the probe.

The first set of coils and the balancing element are configured such that as the probe moves in an axial direction (i.e. parallel to a long axis of the probe) relative to a magnetic marker, the marker induces a sense voltage that shows a single phase change.

The balancing element may generate a sense voltage that wholly offsets, partially offsets, or minimises, a sense voltage induced in the sense coil or coils by the drive coils or coil respectively. Thus, where the first set of coils comprises a first sense coil disposed between a first pair of drive coils, the balancing element may generate a voltage that offsets a voltage induced in the first sense coil from the first pair of drive coils. This means that a net sense voltage measured when a marker moves axially relative to the probe can be wholly, or at least mostly attributed to sense voltage induced from a magnetic marker. The balancing element may also offset any environmental field such as the Earth's magnetic field.

The first set of coils and the balancing element may be housed within a probe housing. The probe housing may have an elongated shape with a long axis. Herein, a direction substantially parallel to the long axis is referred to as an axial direction. The probe housing may be substantially cylindrical in shape. The probe housing may have a stepped cylindrical profile, such that the diameter of the probe housing increases or decreases in a stepwise manner along the length of the probe. The maximum outer diameter of the probe housing may be dependent upon the intended use of the probe. It is often desirable for the probe housing to have a narrow diameter to minimise the size of surgical incision that is required when using the probe. The probe housing may have a maximum outer diameter of between about 3 mm and about 20 mm, or between about 4 mm and about 15 mm, or between about 6 mm and about 10 mm, depending on the intended use of the probe. A probe for use in laparoscopy or robotic surgery may have a smaller diameter of between about 4 mm and about 6 mm, for example. The first set of coils and the balancing element may be disposed within a head of the probe housing proximate to a distal end of the probe.

The first set of coils may be positioned within about 5 mm of the distal end of the probe, preferably within about 3 mm of the distal end of the probe, more preferably within 2 mm from the distal end of the probe. Advantageously, where the first set of coils comprises a first sense coil disposed between a first pair of drive coils, one of the drive coils of the first pair of drive coils may be positioned closest to the distal end to maximise a magnetic driving field produced by the drive coils in the vicinity of a marker.

In operation, the drive coil or coils of the first set of coils may be excited by an AC drive current to produce such a magnetic drive field. When the first set of coils comprises a first pair of drive coils, the first pair of drive coils may be connected in series, and conceptually, can be considered as a single long drive coil that has been divided into two parts (i.e. a 'split' long drive coil). The magnetic drive field extends around first set of coils, and extends outside of the probe housing. The strength of the magnetic drive field is dependent upon the magnitude of the current passing through the or each drive coil, and the number of turns of the or each drive coil. The magnetic drive field may induce a sense response from a magnetic marker proximate to the probe. The magnetic marker may suitably be a ferromagnetic marker, for example, as described in WO2016/193753, WO2019/180580, WO2014/013235.

The sense response from the marker is dependent upon the magnetic field experienced by the marker, which in turn is dependent upon the magnitude of the current passing through the drive coil or coils, and the relative positioning of the marker and the drive coil(s). The sense response is also dependent upon the magnetic permeability of the marker. The sense response from a marker may be detected by the sense coil or coils of the first set of coils, preferably the first sense coil, as a sense voltage. A sense voltage may be processed by a signal processor and the signal processor may generate an output. The output from the signal processor may, for example, be an audible, visual or haptic signal. The output may be interpreted by a user, and used to determine the position of the marker relative to the probe.

The variation in sense voltage arises because the magnetic marker alters the magnetic flux passing through the sense coil or coils of the first set of coils, preferably the first sense coil. The magnetic marker may increase or decrease the magnetic flux that passes through the sense coil or coils of the first set of coils. The sense voltage resulting from the marker is therefore dependent upon whether the magnetic marker increases or decreases the magnetic flux through the sense coil or coils of the first set of coils. The influence that the magnetic marker has on the sense coil or coils is dependent upon the magnetic drive field that the marker has been subjected to. As the probe moves axially relative to a magnetic marker, the marker is subjected to a varying field from the drive coils or coil respectively. The magnetic response induced in the magnetic marker therefore varies and the flux induced in the sense coil or coils varies correspondingly.

The sense coil or coils also detects a sense response from the magnetic drive field originating from the adjacent drive coils or coil of the first set of coils. The sense response from the drive coils or coil, for example the first pair of drive coils, will be significantly larger than the sense response from a marker. In the absence of a balancing element, the response detected in the sense coil or coils would be dominated by the response from the drive coils or coil respectively. The balancing element is used to counteract the sense response from the drive coils or coil of the first set of coils, for example the first pair of drive coils, thereby enabling the sense response from the magnetic marker to be detected. The balancing element may generate a voltage that offsets or minimises the voltage induced in the sense coil or coils of the first set of coils, for example the first sense coil, from the drive coils or coil respectively of the first set of coils, for example the first pair of drive coils.

Mapping the sense voltage from a magnetic marker in around the probe, the sense voltage map shows side lobes that extend in a transverse direction away from the probe (i.e. perpendicular to the long axis of the probe). In three dimensions, the side lobes extend circumferentially around the probe.

For probes according to embodiments of the present disclosure, the sense coil or coils of the first set of coils, for example the first sense coil, may be coupled in series or anti-series, or coiled in the same or an opposite direction to the drive coils or coil of the first set of coils, for example the first pair of drive coils.

Suitably, the first pair of drive coils, where provided, are connected in series. This enables a magnetic drive field of significant magnitude to be generated as the drive field is generated by both coils. By providing a pair of drive coils instead of a single drive coil, each individual coil may be made shorter in length. This may advantageously result in a lower overall inductance and reduced thermal distortions.

The inductance of each drive coil, for example, is proportional to the square of the number of turns in a coil, i.e. $L_{coil} = \frac{\mu_0 \mu_r N^2 A}{l}$, where $L_{coil}$ is the inductance of the coil in Henries (H), $\mu_r$ is the relative permeability of the core, $\mu_0$ is the relative permeability of free space ($4\pi$ x $10^{-7}$ H/m), $N$ is the number of turns on the coil, A is the area of the coil in m$^2$, $r$ is the coil radius in meters, and $l$ is the coil length in meters. By providing a pair of drive coils disposed either side of a sense coil, with each drive coil having fewer turns, the inductance of the coil can be reduced for the same magnetic drive field. Each drive coil may comprise a plurality of radially superimposed layers of wire coiled on top of each other, with each layer having a plurality of turns. Similar considerations apply to a pair of sense coils disposed either side of a drive coil.

By providing a pair of shorter drive coils instead of a single drive coil, good magnetic flux density and a strong drive field may be retained at the distal end of the probe. The axially separated drive coils show less fluctuation in magnetic flux density with axial position than a single long drive coil. The magnetic flux density pattern for axially separated drive coils may show fewer changes in sign in an axial direction, compared to the magnetic flux density pattern of a single long drive coil. Using a pair of shorter drive coils rather than a single long drive coil reduces the extent of the side lobes in the sense voltage that extend in a transverse direction from the side of the probe, when the marker moves axially relative to a marker. As a strong drive field is retained at the distal end of the probe, good probe sensitivity is retained. In some embodiments, the size of the side lobes may be minimised by placing the sense coil next to the at least one drive coil. In embodiments of the present disclosure the distance between the centre of the first coil of the first coil type and the centre of each coil of the first pair of coils of the second type of coil, for example between the centre of the first sense coil and the centre of each drive coil, may be between about 1 mm and about 3 mm.

The first pair of coils of the second coil type, for example drive coils, may comprise two identical coils, i.e. the coils may have the same dimensions as each other and may comprise the same number of turns of wire.

Each drive coil, for example, may have an axial length of between about 0.2 mm and 10 mm, preferably between about 0.5 mm and about 2.5 mm.

The first sense coil may have an axial length of between about 0.5 mm and 6 mm, more preferably between about 0.75 mm and about 2 mm.

Each drive coil may comprise between about 10 and about 150 turns of wire, preferably between about 10 and about 60 turns of wire.

Each drive coil may have an outer radius of between about 0.5 mm and 10mm, more preferably between about 1.5 mm and about 6 mm. The radius of the drive coils may be selected to be appropriate for the diameter of the probe.

The first sense coil may have a mean radius of between about 0.5 mm and 10mm, more preferably between about 1.5 mm and about 6.5 mm. The first sense coil may have a similar mean radius to the mean radius of the drive coils.

The first sense coil may comprise between about 50 and about 1000 turns of wire and preferably about 100 and about 500 turns of wire.

The first sense coil may be formed from wire having a diameter of between about 0.01 and 0.3mm, more preferably between about 0.025 mm and 0.1 mm.

The first sense coil may comprise between about 3 and 20 layers, preferably between about 8 and 10 layers of turns, with each layer having between about 5 and 50 turns, preferably between about 15 and about 20 turns.

The probe housing may be hollow and may define a recess that accommodates the coils. The probe housing may have a wall thickness of between about 0.2 mm and 3mm, more preferably between about 0.5 mm and about 1 mm.

An air gap may be required between the coils and an internal surface of the probe housing to reduce thermal effects. The air gap may be between about 0.2 mm and 3mm, more preferably about 0.5 mm and about 1 mm in the radial dimension.

The total axial distance spanned by the first set of coils and the balancing element may be between about 10 mm and 100mm, more preferably between about 19 mm and about 30 mm. The axial separation between the first sense coil and the balancing element may be between about 3 mm and about 100 mm, preferably between about 12 mm and about 15 mm.

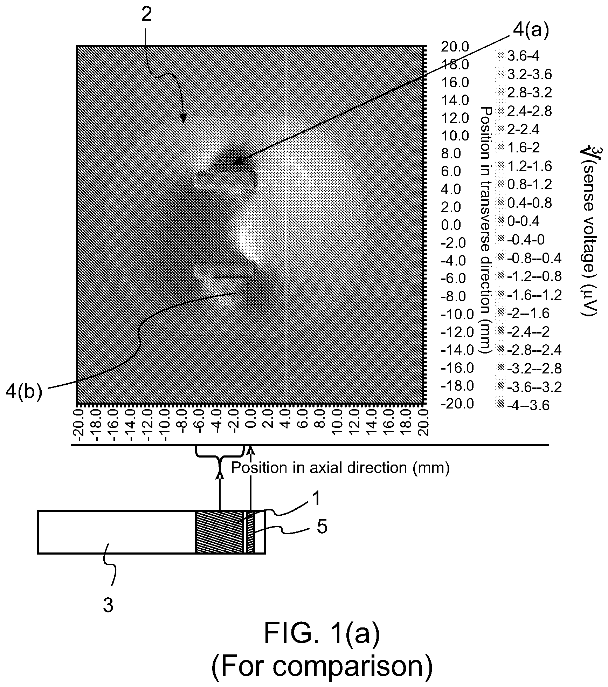

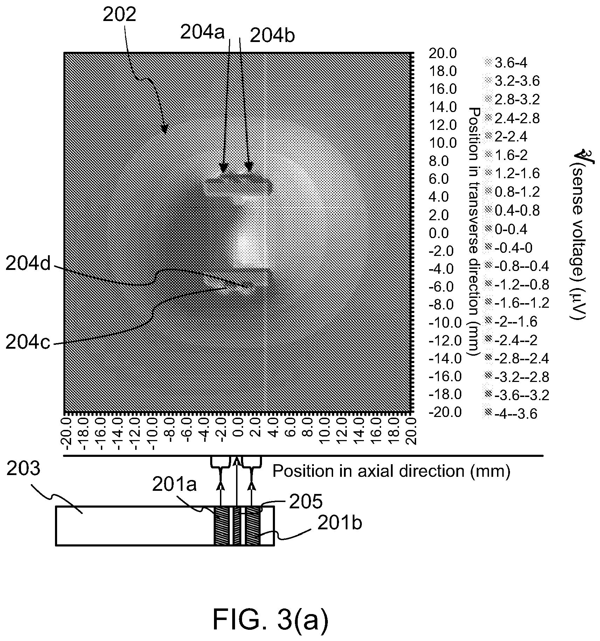

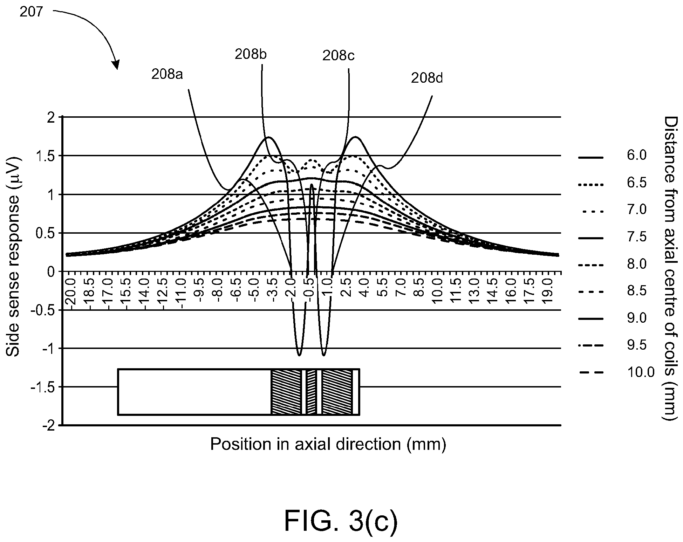

Example plots showing the sense voltage over 2D space and sense response plots for probes comprising comparative drive and sense coil arrangements are shown in Figs. 1(a). 1(b), 2(a) and 2(b). An example plot showing the sense voltage over 2D space and a sense response plot for a probe having a coil arrangement according to an embodiment of the present disclosure are shown in Figs. 3(a) and 3(c). For simplification, in Figs. 1(a)-3(c), the balancing element is not shown.

Fig. 1(a) shows the cubic root of the sense voltage microvolts, $\mu$V, over 2D space 2 for a probe 3 moving axially relative to a marker wherein the probe 3 comprises a long drive coil 1 extending axially along a probe 3. The cubic root of the sense voltage has been plotted owing to the large variation in the sense voltage voltage. The drive coil 1 has a length of about 5 mm. The long drive coil 1 is positioned juxtaposed a sense coil 5 provided towards a distal tip of a probe 3. The drive coil 1 and the sense coil 5 are coiled in opposite directions. Mapping the cubic root of the sense voltage 2 around the drive coil 3 in two dimensions for a probe 3 moving axially relative to a marker, prominent side lobes 4a, 4b can be seen that extend in a transverse direction away from the drive coil 1, beyond an outer housing (not shown) of the probe 3. In three dimensions, these side lobes 4a, 4b form part of a ring extending circumferentially around the probe 3. In operation, as the probe 3 moves relative to a marker in an axial direction (i.e. parallel to the length of the probe 3) outside of the probe housing, the marker is subjected to a varying magnetic flux that reverses direction multiple times. As a result the sense response induced in the sense coil from the marker undergoes a phase flip as the marker moves axially relative to the probe 3.

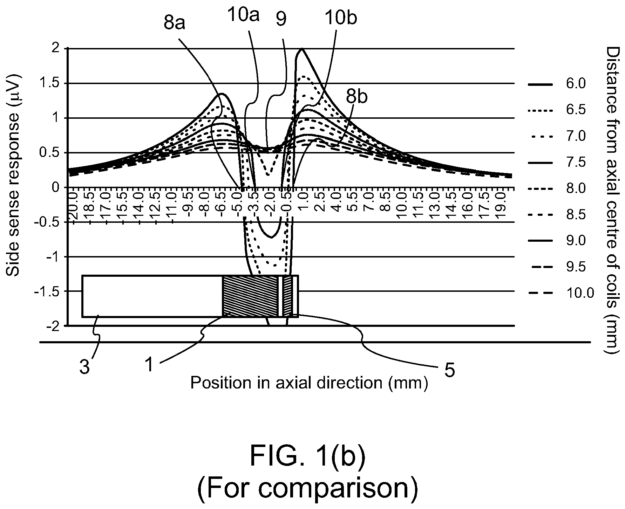

This is shown in Fig. 1(b). Fig. 1(b) plots the side sense response in μV as a function of position in mm, for a marker moving in an axial direction relative to the probe 3 along the length of the probe 3 at different distances from the axial centre of the coils. The side sense response is proportional to the sense voltage induced in the first sense coil. The x-axis indicates the relative position of the magnetic marker along the length of the probe 3, with the probe being superposed on the plots for reference. In this example, taking 0 as being the tip of the probe, the drive coil 1 is a 5 mm drive coil, extending from -1 mm and -6 mm. The sense voltage is dependent upon the strength of the magnetic drive field at the location of the marker (which in turn is dependent upon the length and diameter of the drive coil 1, the current passing through the drive coil 1, and the transverse proximity of the marker to the probe 3). The sense voltage is also dependent on the transverse distance between the marker and the first sense coil 7a (i.e. the distance orthogonal to the length of the probe). The different plots show the sense responses for a marker positioned at different transverse distances from the centre of the probe 3. As the transverse distance from probe 3 increases, the variation in sense response flattens. At relatively small distances from the centre of the probe 3, for example, at 6 mm from the centre of the probe coil, phase flips 8a, 8b, can be seen in the sense response. As the distance from the centre to the marker increases, the sense response flattens. At a distance of 10 mm from the centre of the probe, for example, a ripple 9 can be seen in the sense response as the probe 1 moves axially relative to the marker, but a phase flip is not observed. For the probe 3 shown in Fig. 1(a) that has a single long drive coil, even at relatively large distances from the centre of the drive coil 1, two phase flips may be observed as the probe 3 moves axially relative to the marker. For example, at a distance of 7.5 mm from the centre of the probe phase flips 10a, 10b can be seen in the sense response as the probe 3 moves axially relative to the marker. Therefore even for a relatively large diameter probe, for example, a 15 mm diameter probe, a phase flip may be observed in the sense response as probe 3 moves axially relative to the marker. The sense response is processed by a signal processor and output to a user. Phase flips in the sense response may influence the output, and this may be confusing for a probe user trying to interpret the sense response to determine the position of a marker relative to the probe.

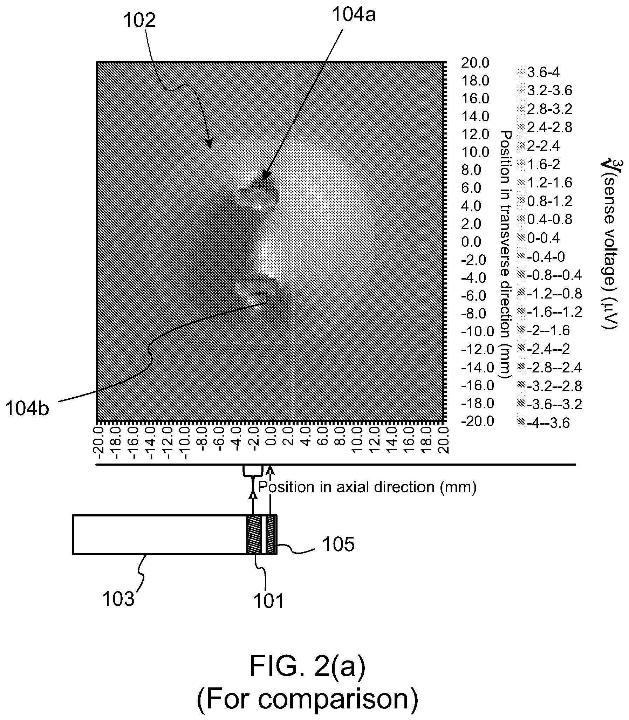

It is recognised that phase flips outside of a probe housing can be avoided simply by using a larger diameter probe housing, such that a larger distance is maintained between the drive coils and a magnetic marker. However, this may be undesirable as it increases the size of surgical incision that is required when a probe is being used. From a surgical perspective, it is preferable for the probe housing diameter to be no larger than is needed to accommodate the coils, the wall thickness of the housing, and an air gap (if used). Phase flips can also be avoided by reducing the length of the drive coil, as shown in Fig. 2(a). This reduces the size of the side lobes in the sense voltage, but also reduces the strength of the magnetic drive field extending from the probe tip. This, in turn, reduces the sensitivity of the probe.

The probe 301 of Fig. 2(a) includes a shorter drive coil 101, having a length of 2.5 mm, positioned adjacent to a sense coil 105. The magnetic flux generated by the drive coil 101 fluctuates less in an axial direction less than for the drive coil 1 shown in Fig. 1(a). The side lobes 104a, 104b in the cubic root sense voltage map 102 extending in a transverse direction away from the probe 103 are less pronounced. The flux extending from the tip of the probe 103 is also less than for the probe 3 of Fig. 1, meaning that the sensitivity of the probe 103, or the distance at which the probe 103 can detect a marker, is reduced.

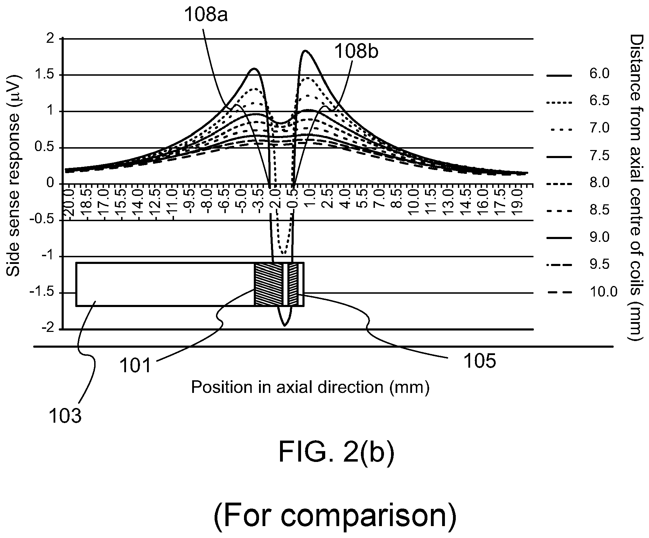

Fig. 2(b) is a plot of the same kind as the plot shown in Fig. 1(b) showing the side sense response as a function of position as a marker moves axially relative to the probe 103 of Fig. 2(a), for markers positioned at different transverse distances from the central axis of the probe. Similarly to the plot 7 for the probe 3 of Fig. 1(b), at short distances from the centre of the probe 103, phase flips 108a, 108b, in the sense response are observed at relatively short distances from the centre of the probe 103 (as shown for the curves at 6 and 6.5 mm from the centre of the drive coil). However, as the side lobes 104a, 104b are less pronounced, the sense response flattens at a shorter transverse distance from the centre of the probe 103. For transverse distances greater than about 7 mm from centre of the probe 103, phase flips are not observed in the sense response. Therefore for larger probe sizes having a diameter of around 15 mm, phase flips will not be observed outside of the probe housing as a marker moves axially along the length of the probe 103. However, the sensitivity of the probe 103 shown in Fig. 2 will be less than the probe 3 of Fig. 1, as the magnetic flux extending from the probe tip is reduced.

Fig. 3(a) shows an example probe 203 and the cubic root of the sense voltage over 2D space 202 generated from a current passing through the drive coils 201a, 201b of a probe 203, according to an embodiment of the present disclosure. For simplicity, the balancing element is not shown. A sense coil 205 is interposed between a first pair of drive coils 201a, 201b. Each drive coil 201a, 201b has a length of about 2.5 mm, so the combined length of the drive coils 201a, 203(b) is similar to the length of the single long drive coil 1 described above with reference to Fig. 1(a). The magnetic flux extending from the tip of the probe 203 generated from a current passing through the drive coils 201a, 201b is similar to the magnetic flux extending from the tip of the probe 3 of Fig. 1(a), thus the sensitivity of the probe 203 of Fig. 3(a) is comparable to the sensitivity of the probe 3 of Fig. 1(a). Advantageously, as the drive coils 203a, 203b are provided as a pair, the total inductance of the drive coils 203a, 203b is lower than for the drive coil 1 of Fig. 1(a), so thermal distortions are reduced.

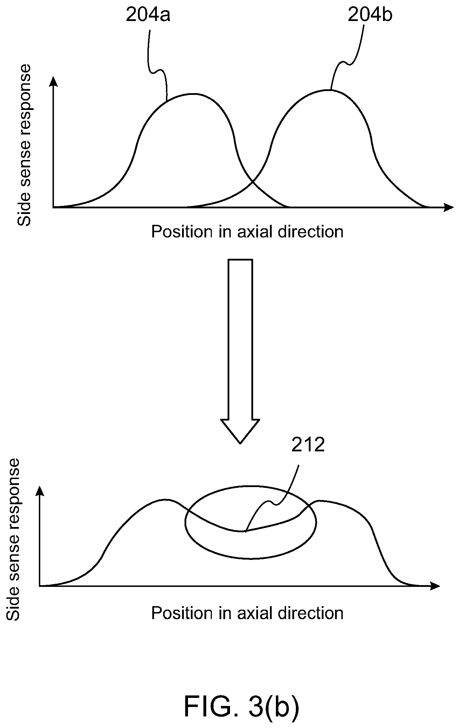

Advantageously, by effectively splitting the single drive coil of Fig. 1(a) into a pair of drive coils 201a, 201b with the same total length, the cubic root of the sense voltage 202 to the side of the probe 203 of Fig. 3(a) is reduced compared to the probe 3 of Fig. 1(a) without sacrificing sensitivity. When the cubic root of the sense voltage is mapped in two dimensions, each drive coil 201a, 201b results in a small pair of side lobes 204a, 204c and 204b, 204d (in three dimensions, the pairs of side lobes 204a, 204c and 204b, 204d form part of two rings extending circumferentially around the probe 203). As there is a small separation between the two drive coils 201a, 201b, the side lobes 204a, 204b and 204c, 204d on the same side of the probe overlap one another. This is shown in Fig. 3(b), and results in a generally flat sense voltage area 212 between the side lobes 204a/204b and 204c/204d. Advantageously, these smaller side lobes 204a, 204b, 204c, 204d, compared to the larger side lobes 4a, 4b, of Fig, 1 are less problematic for a user trying to interpret a marker position using the probe 203, because the side lobes 204a, 204b, 204c, 204d may not extend outside of the probe 203 housing. Furthermore, the overlap between the side lobes 204a, 204b and 204c, 204d on the same side of the probe 203, which results in a generally flat sense voltage area 212 between the side lobes 204a, 204b and 204c, 204d, may enable more accurate positioning of the sense coil 205 between the drive coils 201a, 201b.

Fig. 3(c) is a plot 207 showing the sense response from a probe 203 of Fig. 3(a) as it moves axially relative to a marker, for a marker positioned at a range of different transverse distances from the centre of the probe 203. At short transverse distances from the centre of the coils, as a result of the side lobes, 204a, 204b and 204c, 204d, the sense response shows multiple phase flips 208a, 208b, 208c, 208d as the marker moves axially relative to the probe 203. However, similarly to the plot 107 for the probe 103 of Fig. 2(a), the sense response flattens at larger distances from the centre of the probe 203. For transverse distances greater than about 6.5mm from the centre of the probe, the sense response is generally flat and does not exhibit phase flips in the sensed voltage. This means that even for a smaller probe diameter, for example, a 13 mm diameter probe, phase flips will not be observed outside of the probe housing as the probe 203 moves axially relative to a marker.

The coil arrangement of the probe 203 of Fig. 3(a) may thus enable good sensitivity, as a result of the combined length of the two drive coils 201a, 201b, which is comparable to the sensitivity of the coil configuration of the probe 3 of Fig. 1(a). The coil arrangement of the probe 203 of Fig. 3(a) will result in a lower inductance and therefore less thermal distortion than the coil configuration of the probe 3 of Fig. 1(a), and will result in smaller side lobes. The smaller side lobes of the coil arrangement of the probe 203 of Fig. 3(a) mean that a smaller diameter probe housing can be used, without phase flips occurring outside of the probe housing.

For simplicity, in Figs. 1(a)-3(c), the balancing element that offsets the impact of the drive coils on the sense response has been omitted. The balancing element is configured and arranged to counteract the sense response generated in the first sense coil which can be attributed directly the first pair of drive coils, so that the sense response from the marker can be detected.

While any balancing element may be used which serves in use to generate a voltage that counteracts the voltage induced in the sense coil or coils from the drive coils or coil respectively, in some embodiments, the balancing element may suitably comprise a second set of coils. The second set of coils may comprise a second coil disposed between a second pair of coils. The second coil may be of the same coil type as the first coil. Each of the second pair of coils may be of the same coil type as each of the first pair of coils. Thus, preferably, the second set of coils may comprise a second sense coil interposed between a second pair of drive coils.

In some implementations, the second set of coils may comprise a second coil that is arranged axially proximate the first set of coils and a third coil that is arranged axially remote from the first set of coils, the arrangement being such that the second coil interposes between the first set of coils and the third coil. The second coil may be of the same coil type as the first coil. The third coil may be of the same coil type as each of the first pair of coils. Thus, preferably, the second set of coils may comprise a second sense coil interposed between the first set of coils and a second drive coil.

The second set of coils may be axially separated from the first set of coils, such that the second set of coils is positioned proximally of the probe distal tip. Suitably, the distance between the first set of coils and the balancing element should be such that if a marker is positioned proximate to a distal end of the probe, the response signal from the marker is predominantly due to the magnetic drive field from the first set of coils without being significantly affected by the drive field generated by the balancing element. If a marker is positioned proximate to a second set of coils, the response signal from the marker may be predominantly due to the magnetic drive field from the second set of coils, and the first set of coils may act as the balancing element.

The drive coil or coils of the second set of coils, for example the second pair of drive coils, may suitably be connected in series with the drive coil or coils of the first set of coils.

The drive coil or coils of the second set of coils may be similar to the drive coil or coils respectively of the first set of coils. Thus, for example, the second pair of drive coils may be similar to the first pair of drive coils.

The second set of coils may be substantially identical to the first set of coils.

The sense coil or coils of the second set of coils may be coupled in anti-series with the sense coil or coils of the first set of coils. Alternatively, the sense coil or coils of the second set of coils may be coiled in an opposite direction to the sense coil or coils of the first set of coils, which produces the same effect in use.

Thus, the second sense coil for example may be coupled in series or anti-series with drive coils of the first and second sets of coils and in anti-series with the first sense coil. Alternatively, the second sense coil may be coiled in an opposite direction to the first sense coil, which produces the same effect in use.

Similarly to the drive coil or coils of the first set of coils, the drive coil or coils of the second set of coils may be excited by an AC drive current to produce a magnetic drive field. If both the first pair of drive coils and the second pair of drive coils, for example, are generating a magnetic drive field, sense voltages are induced in both the first sense coil and the second sense coil as a result of the magnetic drive fields from their adjacent drive coils. If the first pair of drive coils and the second pair of drive coils are coiled in the same direction, and if the first sense coil and the second sense coils are coupled in anti-series, or coiled in opposite ways, opposing sense voltages are induced in the first sense coil and the second sense coil from their adjacent drive coils. This means that the sense voltages resulting from the drive coils adjacent to the sense coils cancel each other out. The sense voltage and sense response may therefore be attributed substantially wholly to a sense voltage induced by a magnetic marker proximate to the probe.

The second pair of drive coils, when provided, may comprise two substantially identical coils, i.e. the coils may have substantially identical dimensions and may comprise the same number of turns of wire. Each drive coil may have an axial length of between about 0.2 mm and about 10 mm, preferably between about 0.5 m and about 2.5 mm. The second sense coil may have an axial length of between about 0.5 mm and about 6 mm, preferably between about 0.75 mm and about 2 mm. Each drive coil may comprise between about 10 and about 150 turns of wire, preferably between about 10 and about 60 turns of wire. Each drive coil may have a mean radius of between about 0.5 mm and about 10 mm, preferably between about 1.5 mm and about 6 mm. The radius of the drive coils may be suitable for the diameter of the probe. The second sense coil may have a mean radius of between about 0.5 mm and about 10 mm, preferably between about 1.5 mm and about 6.5 mm. The second sense coil may have a similar mean radius to the mean radius of the drive coils. The second sense coil may comprise between about 50 and about 1000 turns of wire, preferably between about 100 and 500 turns of wire. The distance between the second sense coil and an adjacent drive coil may be between about 0.4 and about 0.8 mm, preferably about 0.7 mm.

The second sense coil may be formed from wire having a diameter of between about 0.01 and 0.3 mm, preferably between about 0.025 and 0.1 mm. The second sense coil may comprise between about 8 and about 10 layers, with each layer having between 5 and 50 turns, preferably between about 15 and 20 turns.

The total axial length spanned by the first set of coils and the second set of coils, including spaces intermediate adjacent coils, may be between about 10 mm and about 100 mm, preferably between about 19 mm and about 30 mm. The axial separation between the midpoint of the first coil of the first coil type, for example the first sense coil, and the midpoint of the balancing element may be between about 3 mm and about 100 mm, preferably between about 12 mm and about 15 mm. The axial separation between the first sense coil, for example, and the second sense coil may be at least 10 mm to reduce the risk of the second sense coil sensing a magnetic marker and interfering with the sensing signal from the first sense coil.

In some implementations of the present disclosure, as mentioned above, the second set of coils may comprise a second coil of the same coil type as the first coil that is arranged axially proximate the first set of coils, and a third coil of the same coil type as each of the first pair of coils that is arranged axially remote from the first set of coils, the arrangement being such that the second coil interposes between the first set of coils and the third coil.

Thus, the probe may for example comprise a second sense coil and a drive coil arranged distally of the second sense coil, but no additional drive coil proximally of the second sense coil. Instead, the proximal drive coil that is furthest from the probe tip of the first pair of drive coils may serve as a proximal drive coil for the first pair of drive coils and also as a distal drive coil for a coil arrangement that is equivalent to a second pair of drive coils of the kind described above. Thus, the drive coil that is furthest from the probe tip may serve a role in both the first and second sets of coils, allowing one drive coil to be omitted as compared with the above-described arrangement. A similar arrangement would obtain where the first set of coils comprises a single drive coil between two sense coils: the second set of coils may comprise a single distal drive coil and at least one proximal sense coil, whereby the distal drive coil interposes between a proximal one of the sense coils of the first set of coils and the at least one proximal sense coil of the second set.

Conversely, in some implementations, the second set of coils may comprise a single drive coil, proximal to the sense coil. The second set of coils may be axially separated from the first set of coils, such that the second set of coils is positioned proximally of the probe distal tip.

According to a third aspect, the present disclosure provides a detection system for locating a magnetic marker. The detection system comprises a probe. The probe may include any of the features outlined above. The system comprises a magnetic field generator arranged to drive an alternating magnetic field through one or more drive coils of a first set of coils, preferably a first pair of drive coils, and the balancing element. The system suitably comprises at least one detector arranged to receive a signal indicative of a sense voltage. In some embodiments, the detection system may further comprise one or more magnetic markers for inducing a sense voltage in one or more sense coils, for example the first sense coil. The system may comprise a signal processor for processing the sense voltage.

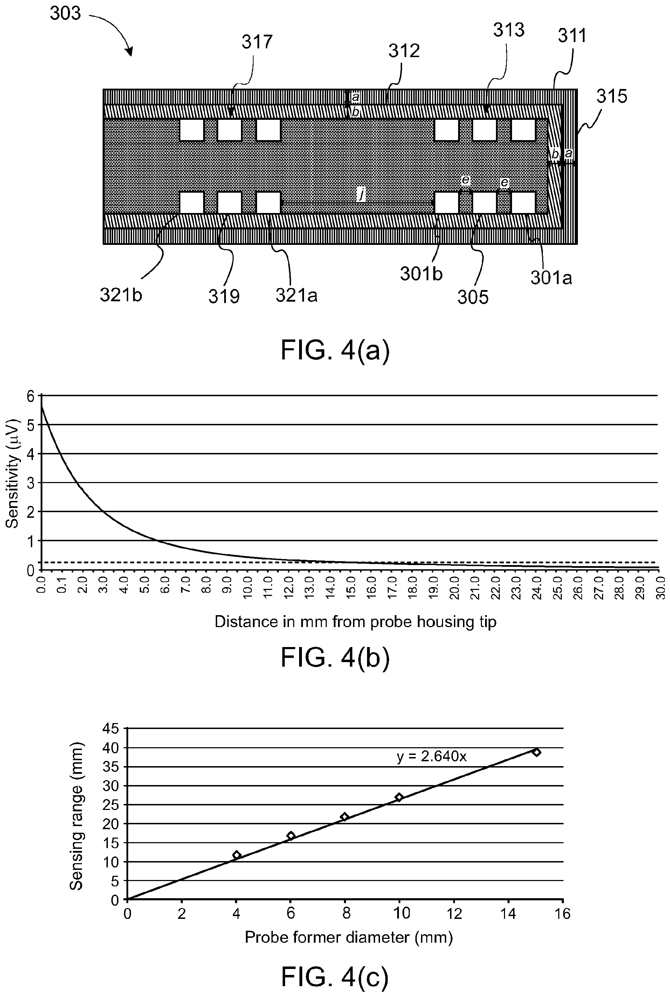

Fig. 4(a) shows a schematic cross-sectional drawing of a distal end portion of a probe 303 according to an embodiment of the present disclosure. The probe 303 comprises a substantially cylindrical probe housing 311, having a diameter of 10 mm. In other embodiments, the housing may comprise sections of different diameters and/or have a maximum outer diameter in the range of about 3 mm to about 20 mm and may be dependent upon the intended use of the probe. The relatively small probe diameter of the probe may facilitate improved visualisation of a surgical site of investigation and better accuracy in identifying the marker location and may reduce the size of an incision required to be made by a surgeon when using the probe.

The probe housing 311 has a thickness of 1 mm, indicated by the letter 'a'. In other embodiments, the probe housing may have a thickness of between about 0.5 mm and about 2 mm. There is an air gap of approximately 1mm between an inner surface 312 of the probe housing 311 and the coils within the housing 311, indicated by letter 'b', which help avoid thermal drift. For smaller diameter probes for example, having an outer diameter of between about 4 mm and about 6 mm, an air gap may not be required between the inner surface of the probe housing and the coils. Instead, a coating may be applied to the interior surface of the housing. Advantageously, this may allow for further reduction in the probe size, and enable less material to be used within the probe. This may enable a constant probe temperature to be more readily achieved, reducing thermal drift.

The probe housing 311 defines a recess which accommodates a first set of coils 313 proximate to a distal sensing end 315 of the probe 303, and a second set of coils 317 which are axially separated from the first set of coils 313 along a length of the probe 303. There is a gap of about 2 mm (indicated by letter 'b') between the inner surface of the probe housing at the probe tip and a former material surrounding the coils 313, 317.

The first set of coils 313 comprises a first sense coil 305, interposed between a first pair of drive coils 301a, 301b. The second set of coils 317 comprises a second sense coil 319, interposed between a second pair of drive coils 321a, 321b. The four drive coils 301a, 301b, 321a, 321b are all coiled in the same direction (i.e. in series) and are connected to an AC current source (not shown). In operation, the drive coils 301a, 301b, 321a, 321b generate a magnetic drive field, which induces a magnetic moment in a marker (not shown) proximate to the probe 303.

The axial separation between the first set of coils and the second set of coils is 12.6 mm, indicated as letter 'j'. Separating the first set of coils 313 and the second set of coils 317 such that they span a significant length of the distal end portion of the probe 303 may increase the sensitivity or sensing distance of the probe 303, such that a marker can be detected at a greater distance from the probe tip 315. Each drive coil 301a, 301b, 321a, 321b has a length of about 2mm and a mean radius of about 4 mm. The four drive coils 301a, 301b, 321a, 321b are substantially identical to one another, with each drive coil 301a, 301b, 321a, 321b comprising 6 radially superposed layers, each layer having 6 turns of 330 $\mu$m diameter wire (leading to 36 turns in total). By using pairs of relatively short drive coils 301a, 301b, 321a, 321b rather than a single long drive coil to generate magnetic drive fields, the inductance of the drive coils 301a, 301b, 321a, 321b is reduced, and a suitable drive field can be generated using a lower voltage. This may reduce heating within the probe 303.

The sense coils 305, 319 are coiled in opposite directions, but are otherwise substantially identical to one another. Each sense coil 305, 319 comprises 9 radially superposed layers, each layer having 18 turns, leading to 162 turns per coil in total.

The first sense coil 305 and second sense coil 319 both have a mean radius of about 4.5 mm and a length of about 2 mm. Each sense coil 305, 319 is formed of 110 $\mu$m diameter wire. The spacing between each sense coil 305, 319 and its adjacent drive coils is about 0.7 mm, indicated as letter 'e'.

As each sense coil 305; 319 is disposed between two drive coils 301a, 301b; 321a, 321b, and as the sense coils 305, 319 are coiled in opposite directions from one another, the drive coils 301a, 301b, 321a, 321b generate opposing signals in the sense coils in use. The direct effects of the drive coils 301a, 301b, 321a, 321b on the sense coils 305, 319 can therefore be cancelled out during processing of the sensed voltage signals.

As disclosed here, in a variant of the present embodiment, each of the drive coils 301a, 301b; 321a, 321b and sense coils 305, 319 may be interchanged with a coil of the other type (i.e. a sense or drive coil respectively), so that each of the first and second sets of coils 313, 317 comprises a single drive coil interposed between a pair of sense coils, with the drive coils of the first and second sets 313, 317 being wound in mutually opposite directions or connected in anti-series, and the pairs of sense coils of the first and second sets of coils being wound in the same direction and connected in series.

The probe 303 of the present embodiment is capable of detecting a magnetic marker at a distance, or sensing range of up to about 15 mm from the tip 315 of the probe 303. This is shown in Fig. 4(b), which plots sensitivity (measured as the cubic root of the sensed voltage in μV) as a function of distance in mm from the tip of the probe. Below 0.2 μV (indicated by the dashed line), corresponding to a sense voltage of 8nV, the signal is considered too weak for accurate detection.

Fig. 4(c) is a plot showing how sensing range (in mm) varies with probe former diameter (in mm) for probes according to embodiments of the present disclosure. The former is provided within the probe housing, and the probe former diameter will be dictated, at least in part, by the diameter and the spacing of the coils.

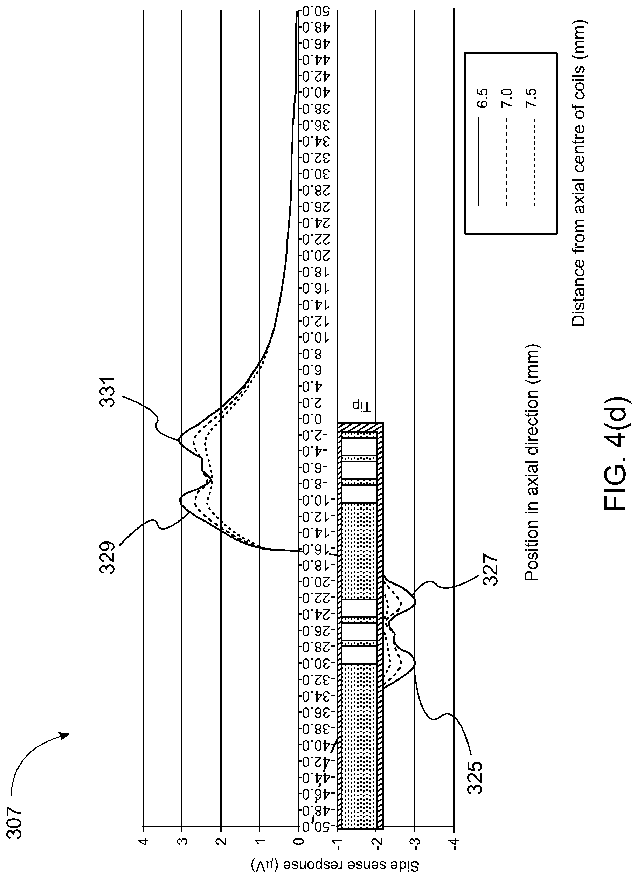

When the probe moves axially relative to the marker, the magnetic field from the first and second pair of drive coils 301a, 301b, 321a, 321b induce a magnetic moment in the marker. The magnetic moment induced in the marker induces a sense voltage in the sense coil 305 proximate to the probe tip 315. The drive coils 301a, 301b, 321a, 321b also induce equal and opposite sense voltages in the sense coils 305, 319. The net sense voltage from the two sense coils 305, 319 can thus be attributed substantially wholly to the sense response from the marker, because the sense voltages from the drive coils cancel each other out. The variation in sense voltage as the probe 303 moves axially relative to the marker is shown in Fig. 4(d).

The plot 307 shown in Fig. 4(d) shows the side sense response from the sense coils 305, 319 of the probe 303 shown in Fig 4(a), as the probe 303 moves axially relative to the marker. The sense response is plotted for a marker at transverse distances of 6.5 mm, 7 mm, and 7.5 mm from the centre of the probe. The x-axis indicates the relative position of the magnetic marker along the length of the probe 303, with the probe 303, being superposed on the plot 307 for reference. Each pair of drive coils 301a, 301b; 321a, 321b results in two corresponding side lobes in the sense voltage that extend circumferentially around the probe 303. For a marker moving axially relative to the probe 303, at a constant transverse distance from the probe 303, the four drive coils 301a, 301b; 321a, 321b give rise to four peaks 325, 327, 329, 331 in the sense response, corresponding to the side lobes. As the spacing between the drive coils within each pair is small (around 5.5 mm between the drive coil centres), the side lobes resulting from the drive coils within a pair overlap one another as described above, and the peaks partially overlap as shown in Fig. 4(d).

As explained above with reference to Fig. 3(a), the coil arrangement of Fig. 4(a), may result in relatively small side lobes as compared with the coil configuration of Fig. 1(a), as the drive coil configuration reduces the magnetic flux extending from the side of the probe 303, whilst retaining reasonably strong magnetic flux extending from the probe tip 315. The side lobes in the sense response are further diminished as a result of the small spacing of around 3.5 mm between the centres of each drive coil 301a, 301b and 321a, 321b within a pair. As a result of these smaller side lobes, outside the probe housing (i.e. at a transverse distance of greater than 5 mm from the centre of the coils in this embodiment), a phase flip is not observed in the sense response either the first set of coils or the second set of coils pass alongside a marker. A single phase flip is observed as the marker moves relative to the probe 303 in the region intermediate the first and second set of coils. This contrasts to the sense response that would be observed for a probe having the coil configuration shown in Fig. 1(a), which would show multiple phase flips outside the probe housing for a probe moving axially relative to a marker. The probe 303 of Fig. 4(a), therefore advantageously shows a strong sensitivity and low thermal distortions and shows only a single phase flip as the probe moves axially relative to the marker, as a results of the short pairs of drive coils, and the small separation between the drive coils.

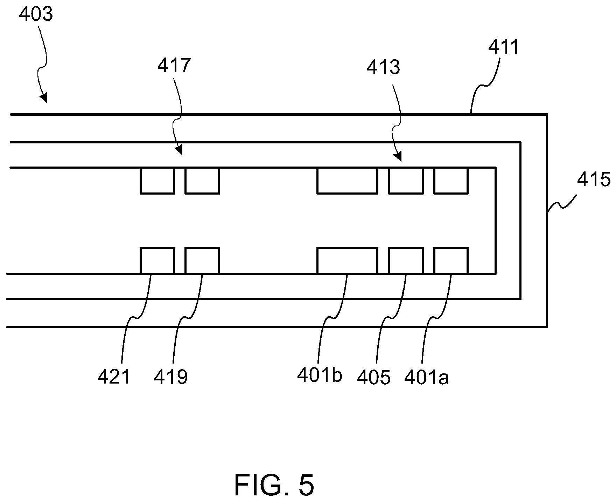

Fig. 5 shows a schematic longitudinal sectional drawing of a distal end portion of a probe 403 according to a different implementation of the present disclosure. The probe 403 is similar to the probe 303 of Fig. 4(a), comprising a substantially cylindrical probe housing 411. The probe housing 411 defines an interior recess which accommodates a first set of coils 413 proximate to a distal sensing end 415 of the probe 403. The first set of coils 413 comprises a first sense coil 405, interposed between a first pair of drive coils 401a, 401b. A second set of coils 417 is axially separated from the first set of coils 413 along the length of the probe 403. In this implementation, the second set of coils 417 comprises a second sense coil 419 and a drive coil 421 arranged proximally of the second sense coil 419, but no drive coil distally of the second sense coil 419, intermediate the first set of coils 413.

In the embodiment shown in Fig. 4(a), the first pair of drive coils 301a, 301b and the second pair of drive coils 321a, 321b are all coiled in the same direction (i.e. in series) and are connected to an AC current source (not shown). The sense coils 305, 319 are coiled in opposite directions, but are otherwise substantially identical to one another.

In the embodiment shown in Fig. 5, the first set of coils 413 comprises a pair of drive coils 401a, 401b, but the second set of coils 417 comprises only a single drive coil 421. All of the drive coils 401a, 401b, 421 are coiled in the same direction (i.e. in series) and are connected to an AC current source (not shown). In operation, at least the drive coils 401a, 401b generate a magnetic drive field, which induces a magnetic moment in a marker (not shown) proximate to the probe 403. The proximal drive coil 401b of the first set of coils 413 that is further away from the edge of the probe tip performs both the function of the corresponding proximal drive coil 301b in in Fig. 4(a), and additionally, and performs the function of the distal drive coil 321a of the second set of drive coils 317 that is closer to the probe tip, as shown in Fig. 4(a).

The drive coils 401a, 401b, 421 are all coiled in the same direction (i.e. in series) and are connected to an AC current source (not shown). In operation, the drive coils 401a, 401b, 421 generate a magnetic drive field, which induces a magnetic moment in a marker (not shown) proximate to the probe 403. The outer drive coils, 401a, 421 (i.e., the drive coil 401a closest to the sensing end of the probe 415 and the single drive coil 421 that forms part of the second set of coils 417) each comprise 10 radially superimposed layers, each layer having 10 turns of 330 μm diameter wire (leading to 100 turns in total). The proximal coil 401b of the first set of coils 413 (i.e., the central drive coil) comprises 7 radially superimposed layers, each layer having 7 turns of 330 μm diameter wire (leading to 49 turns in total). The proximal coil 401b has fewer turns than the each of the outer drive coils, 401a, 421, and the proximal coil 401b has fewer turns than the sum of the central drive coils 321a, 301b of the configuration shown in Fig. 4(a) (the central drive coils 321a, 301b of Fig. 4(a) have a total of 72 turns), The reduction in turns of the proximal coil 401b compared to the outermost drive coils, 401a, 421 helps to maintain a constant detection signal along the axial length of the probe 403, and may facilitate easier manufacturing of the probe.

Similarly to the probe 303 of Fig. 4(a), the sense coils 405, 419 of Fig. 5 are coiled in opposite directions, but are otherwise substantially identical to one another. Each sense coil 405, 419 comprises 9 radially superposed layers, each layer having 18 turns, leading to 162 turns per coil in total. As each sense coil 405; 419 is disposed between two adjacent ones of the drive coils 401a, 401b; 421 and the sense coils 405, 419 are coiled in opposite directions from one another, the drive coils 401a, 401b, 421 generate opposing signals in the sense coils 405, 419 in use. The direct effects of the drive coils 401a, 401b, 421 on the sense coils 405, 419 can therefore be substantially cancelled out during processing of the sensed voltage signals.

As with the probe 303 of Fig. 4(a), in a variant of the present embodiment, each of the drive coils 401a, 401b; 421 and sense coils 405, 419 may be interchanged with a coil of the other type (i.e. a sense or drive coil respectively), so that the first set of coils 413 comprises a single drive coil interposed between a pair of sense coils, and the second set of coils 417 comprises a single drive coil and a single sense coil, with the single drive coil being interposed between the single sense coil and the first set of coils. In such case, the drive coils of the first and second sets 413, 417 may be wound in mutually opposite directions or connected in anti-series, while the sense coils of the first and second sets of coils may all be wound in the same direction and connected in series.

Although aspects of the present disclosure have been described with reference to particular embodiments, it is to be understood that these embodiments are merely illustrative of the principles and applications of the disclosure. It is therefore to be understood that numerous modifications may be made to the illustrative embodiments and that other arrangements may be devised without departing from the scope of the invention as defined by the appended claims.

It will be appreciated by those of ordinary skill in the art that features of these example embodiments may be combined in other embodiments that fall within the scope of the appended claims.

While various details have been set forth in the foregoing description, it will be appreciated that the various aspects of the techniques for operating a diagnostic and/or surgical guidance system suitable for identifying, localizing, tracking, and detecting position of one or more implanted markers may be practiced without these specific details. One skilled in the art will recognize that the herein described components (e.g., operations), devices, objects, and the discussion accompanying them are used as examples for the sake of conceptual clarity and that various configuration modifications are contemplated. Consequently, as used herein, the specific exemplars set forth and the accompanying discussion are intended to be representative of their more general classes. In general, use of any specific exemplar is intended to be representative of its class, and the non-inclusion of specific components (e.g., operations), devices, and objects should not be taken limiting.

Further, while several forms have been illustrated and described, it is not the intention of the applicant to restrict or limit the scope of the appended claims to such detail. Numerous modifications, variations, changes, substitutions, combinations, and equivalents to those forms may be implemented and will occur to those skilled in the art without departing from the scope of the present invention, which is defined by the appended claims.

Whilst in the foregoing description, integers or elements are mentioned which have known obvious or foreseeable equivalents, then such equivalents are herein incorporated as if individually set forth. Reference should be made to the claims for determining the true scope of the present disclosure, which should be construed as to encompass any such equivalents. It will also be appreciated by the reader that integers or features of the disclosure that are described as advantageous, convenient or the like are optional, and do not limit the scope of the independent claims. Moreover, it is to be understood that such optional integers or features, whilst of possible benefit in some embodiments of the disclosure, may not be desirable and may therefore be absent in other embodiments.

The phrase "and/or," as used herein in the specification and in the claims, should be understood to mean "either or both" of the elements so conjoined, i.e., elements that are conjunctively present in some cases and disjunctively present in other cases.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

The terms "approximately" and "about" may be used to mean within ±20% of a target value in some embodiments, within ±10% of a target value in some embodiments, within ±5% of a target value in some embodiments, and yet within ±2% of a target value in some embodiments. The terms "approximately" and "about" may include the target value.

In the claims, as well as in the specification above, all transitional phrases such as "comprising," "including," "carrying," "having," "containing," "involving," "holding," "composed of," and the like are to be understood to be open-ended, i.e., to mean including but not limited to. The transitional phrases "consisting of" and "consisting essentially of" shall be closed or semi-closed transitional phrases, respectively.

Where a range or list of values is provided, each intervening value between the upper and lower limits of that range or list of values is individually contemplated and is encompassed within the disclosure as if each value were specifically enumerated herein. In addition, smaller ranges between and including the upper and lower limits of a given range are contemplated and encompassed within the disclosure. The listing of exemplary values or ranges is not a disclaimer of other values or ranges between and including the upper and lower limits of a given range.

Embodiments disclosed herein may be embodied as a system, method or computer program product. Accordingly, embodiments may take the form of an entirely hardware embodiment, an entirely software embodiment (including firmware, resident software, microcode, etc.) or an embodiment combining software and hardware aspects that may all generally be referred to herein as a "circuit," "module," or "system." Furthermore, embodiments may take the form of a computer program product embodied in one or more computer readable medium(s) having computer readable program code embodied thereon.

**Claims**

1. A probe (203) for surgical use, for sensing a magnetic marker, the probe (203) comprising:

   a first set of coils comprising a first coil of a first coil type disposed between a first pair of coils of a second coil type that are connected in series, wherein the first coil type is either a sense coil or a drive coil, and the second coil type is respectively either a drive coil or a sense coil; and
a balancing element which is axially separated from the first set of coils along a length of the probe (203), wherein the balancing element is configured and arranged to generate a sense voltage that wholly offsets, partially offsets, or minimises, a sense voltage induced in the sense coil or coils by the drive coils or coil respectively, and **CHARACTERISED IN THAT** the balancing element comprises a second set of coils comprising a second coil disposed between a second pair of coils; wherein the second coil is of the same coil type as the first coil and each of the second pair of coils is of the same coil type as each of the first pair of coils.

2. A probe (203) for surgical use, for sensing a magnetic marker, the probe (203) comprising:

   a first set of coils comprising a first coil of a first coil type disposed between a first pair of coils of a second coil type that are connected in series, wherein the first coil type is either a sense coil or a drive coil, and the second coil type is respectively either a drive coil or a sense coil; and
   a balancing element which is axially separated from the first set of coils along a length of the probe (203), wherein the balancing element is configured and arranged to generate a sense voltage that wholly offsets, partially offsets, or minimises, a sense voltage induced in the sense coil or coils by the drive coils or coil respectively, and

   **CHARACTERISED IN THAT** the balancing element comprises a second set of coils comprising a second coil that is arranged axially proximate the first set of coils and a third coil that is arranged axially remote from the first set of coils, the arrangement being such that the second coil interposes between the first set of coils and the third coil; wherein the second coil is of the same coil type as the first coil and the third coil is of the same coil type as each of the first pair of coils.

3. A probe (203) according to any of claims 1 or claim 2, wherein the first coil type is a sense coil (205) and the second coil type is a drive coil (201a, 201b).

4. A probe (203) according to any preceding claim, wherein the total axial distance spanned by the first set of coils and the balancing element is between 19 mm and 30 mm.

5. A probe (203) according to any preceding claim, wherein the or each drive coil has an axial length of between 0.5 mm and 2.5 mm.

6. A probe (203) according to any preceding claim, wherein the or each sense coil has an axial length of between 0.75 mm and 2 mm.

7. A probe (203) according to any preceding claim, wherein the axial separation between the first coil and the balancing element is between 12 mm and 15 mm.

8. A probe (203) according to any preceding claim, wherein each drive coil has a mean radius of between 1.5 mm and 6 mm.

9. A probe (203) according to any preceding claim, the or each sense coil has an outer radius of between 1.5 mm and 6.5 mm.

10. A probe (203) according to any preceding claim, wherein the or each drive coil comprises between 10 and 60 turns of wire.

11. A probe (203) according to any preceding claim, wherein the or each sense coil comprises between 100 and 500 turns of wire.

12. A probe (203) according to any preceding claim, wherein the drive coils of the first and second sets of coils are connected in series.

13. A probe (203) according to any preceding claims, wherein the sense coil or coils of the second set of coils are coupled in anti-series with the sense coil or coils of the first set of coils.

14. A probe (203) according to any preceding claim, wherein the sense coil or coils of the second set of coils are coiled in an opposite direction to the sense coil or coils of the first set of coils.

15. A probe (203) according to any preceding claim, wherein the first set of coils and the balancing element are housed within a probe housing, and wherein the probe housing has a maximum outer diameter of between 4 mm and 15 mm.

16. A detection system for locating a magnetic marker, the system comprising:

a probe (203) for surgical use as claimed in any of claims 1-15;
a magnetic field generator arranged to drive an alternating magnetic field through the drive coil or coils of the first set of coils and the balancing element; and
at least one detector arranged to receive a signal indicative of a sense voltage.

**Patentansprüche**

1. Sonde (203) für den chirurgischen Einsatz, zum Erfassen eines magnetischen Markers, wobei die Sonde (203) aufweist:

einen ersten Spulensatz, der eine erste Spule eines ersten Spulentyps, die zwischen einem ersten Paar von Spulen eines zweiten Spulentyps, die in Reihe geschaltet sind, angeordnet ist, aufweist, wobei der erste Spulentyp entweder eine Erfassungsspule oder eine Antriebsspule ist und der zweite Spulentyp jeweils entweder eine Antriebsspule oder eine Erfassungsspule ist; und
ein Abgleichelement, das entlang einer Länge der Sonde (203) axial von dem ersten Spulensatz getrennt ist, wobei das Abgleichelement dazu ausgebildet und angeordnet ist, eine Erfassungsspannung zu erzeugen, die eine in der Erfassungsspule oder den Erfassungsspulen durch die Antriebsspulen oder Antriebsspule induzierte Erfassungsspannung vollständig ausgleicht, teilweise ausgleicht oder minimiert, und

**DADURCH GEKENNZEICHNET, DASS** das Abgleichelement einen zweiten Spulensatz, der eine zweite Spule, die zwischen einem zweiten Paar von Spulen angeordnet ist, aufweist, aufweist; wobei die zweite Spule vom gleichen Spulentyp ist wie die erste Spule und jede Spule des zweiten Spulenpaares vom gleichen Spulentyp ist wie jede Spule des ersten Spulenpaares.

2. Sonde (203) für den chirurgischen Einsatz, zum Erfassen eines magnetischen Markers, wobei die Sonde (203) aufweist:

einen ersten Spulensatz, der eine erste Spule eines ersten Spulentyps, die zwischen einem ersten Paar von Spulen eines zweiten Spulentyps, die in Reihe geschaltet sind, angeordnet ist, aufweist, wobei der erste Spulentyp entweder eine Erfassungsspule oder eine Antriebsspule ist und der zweite Spulentyp jeweils entweder eine Antriebsspule oder eine Erfassungsspule ist; und
ein Abgleichelement, das entlang einer Länge der Sonde (203) axial von dem ersten Spulensatz getrennt ist, wobei das Abgleichelement dazu ausgebildet und angeordnet ist, eine Erfassungsspannung zu erzeugen, die eine in der Erfassungsspule oder den Erfassungsspulen durch die Antriebsspulen oder Antriebsspule induzierte Erfassungsspannung vollständig ausgleicht, teilweise ausgleicht oder minimiert, und

**DADURCH GEKENNZEICHNET, DASS** das Abgleichelement einen zweiten Spulensatz, der eine zweite Spule, die axial proximal zu dem ersten Spulensatz angeordnet ist, und eine dritte Spule, die axial distal von dem ersten Spulensatz angeordnet ist, aufweist, aufweist, wobei die Anordnung derart ist, dass die zweite Spule zwischen dem ersten Spulensatz und der dritten Spule liegt; wobei die zweite Spule vom gleichen Spulentyp ist wie die erste Spule und die dritte Spule vom gleichen Spulentyp ist wie jede Spule des ersten Spulenpaares.

3. Sonde (203) nach einem der Ansprüche 1 oder 2, wobei der erste Spulentyp eine Erfassungsspule (205) ist und der zweite Spulentyp eine Antriebsspule (201a, 201b) ist.

4. Sonde (203) nach einem der vorhergehenden Ansprüche, wobei der gesamte axiale Abstand, der von dem ersten Spulensatz und dem Abgleichelement überspannt wird, zwischen 19 mm und 30 mm beträgt.

5. Sonde (203) nach einem der vorhergehenden Ansprüche, wobei die oder jede Antriebsspule eine axiale Länge zwischen 0,5 mm und 2,5 mm aufweist.

6. Sonde (203) nach einem der vorhergehenden Ansprüche, wobei die oder jede Erfassungsspule eine axiale Länge zwischen 0,75 mm und 2 mm aufweist.

7. Sonde (203) nach einem der vorhergehenden Ansprüche, wobei der axiale Abstand zwischen der ersten Spule und dem Abgleichelement zwischen 12 mm und 15 mm beträgt.

8. Sonde (203) nach einem der vorhergehenden Ansprüche, wobei jede Antriebsspule einen mittleren Radius zwischen 1,5 mm und 6 mm aufweist.

9. Sonde (203) nach einem der vorhergehenden Ansprüche, wobei die oder jede Erfassungsspule einen Außenradius zwischen 1,5 mm und 6,5 mm aufweist.

10. Sonde (203) nach einem der vorhergehenden Ansprüche, wobei die oder jede Antriebsspule zwischen 10 und 60 Drahtwindungen aufweist.

11. Sonde (203) nach einem der vorhergehenden Ansprüche, wobei die oder jede Erfassungsspule zwischen 100 und 500 Drahtwindungen aufweist.

12. Sonde (203) nach einem der vorhergehenden Ansprüche, wobei die Antriebsspulen des ersten und zweiten Spulensatzes in Reihe geschaltet sind.

13. Sonde (203) nach einem der vorhergehenden Ansprüche, wobei die Erfassungsspule oder -spulen des zweiten Spulensatzes in Gegenserie mit der Erfassungsspule oder den Erfassungsspulen des ersten Spulensatzes geschaltet sind.

14. Sonde (203) nach einem der vorhergehenden Ansprüche, wobei die Erfassungsspule oder -spulen des zweiten Spulensatzes in einer entgegengesetzten Richtung zu der Erfassungsspule oder den Erfassungsspulen des ersten Spulensatzes gewickelt sind.

15. Sonde (203) nach einem der vorhergehenden Ansprüche, wobei der erste Spulensatz und das Abgleichelement in einem Sondengehäuse untergebracht sind, und wobei das Sondengehäuse einen maximalen Außendurchmesser zwischen 4 mm und 15 mm aufweist.

16. Detektionssystem zur Ortung eines magnetischen Markers, wobei das System aufweist:

eine Sonde (203) für den chirurgischen Einsatz nach einem der Ansprüche 1-15;
einen Magnetfeldgenerator, der angeordnet ist, ein alternierendes Magnetfeld durch die Antriebsspule oder -spulen des ersten Spulensatzes und das Abgleichelement anzutreiben; und mindestens einen Detektor, der angeordnet ist, ein Signal zu empfangen, das eine Erfassungsspannung anzeigt.

## Revendications

1. Sonde (203) à usage chirurgical, pour détecter un marqueur magnétique, la sonde (203) comprenant :

un premier ensemble de bobines comprenant une première bobine d'un premier type de bobine disposée entre une première paire de bobines d'un second type de bobine qui sont connectées en série, dans laquelle le premier type de bobine est soit une bobine de détection, soit une bobine d'entraînement, et le second type de bobine est respectivement soit une bobine d'entraînement, soit une bobine de détection ; et
un élément d'équilibrage qui est séparé axialement du premier ensemble de bobines sur une longueur de la sonde (203), dans laquelle l'élément d'équilibrage est configuré et agencé pour générer une tension de détection qui compense entièrement, compense partiellement ou minimise une tension de détection induite dans la ou les bobines de détection par la ou les bobines d'entraînement respectivement, et **CARACTÉRISÉE EN CE QUE** l'élément d'équilibrage comprend un deuxième ensemble de bobines comprenant une deuxième bobine disposée entre une seconde paire de bobines ; dans laquelle la deuxième bobine est du même type de bobine que la première bobine et chacune de la seconde paire de bobines est du même type de bobine que chacune de la première paire de bobines.

2. Sonde (203) à usage chirurgical, pour détecter un marqueur magnétique, la sonde (203) comprenant :

un premier ensemble de bobines comprenant une première bobine d'un premier type de bobine disposée entre une première paire de bobines d'un second type de bobine qui sont connectées en série, dans laquelle le premier type de bobine est soit une bobine de détection, soit une bobine d'entraînement, et le second type de bobine est respectivement soit une bobine d'entraînement, soit une bobine de détection ; et
un élément d'équilibrage qui est séparé axialement du premier ensemble de bobines sur une longueur de la sonde (203), dans laquelle l'élément d'équilibrage est configuré et agencé pour générer une tension de détection qui compense entièrement, compense partiellement ou minimise une tension de détection induite dans la ou les bobines de détection par la ou les bobines d'entraînement respectivement, et **CARACTÉRISÉE EN CE QUE** l'élément d'équilibrage comprend un second ensemble de bobines comprenant une deuxième bobine qui est agencée axialement à proximité du premier ensemble de bobines et une troisième bobine qui est agencée axialement à distance du premier ensemble de bobines, l'agencement étant tel que la deuxième bobine s'interpose entre le premier ensemble de bobines et la troisième bobine ; dans laquelle la deuxième bobine est du même type de bobine que la première bobine et la troisième bobine est du même type de bobine que chacune de la première paire de bobines.

3. Sonde (203) selon l'une quelconque des revendications 1 ou 2, dans laquelle le premier type de bobine est une bobine de détection (205) et le second type de bobine est une bobine d'entraînement (201a, 201b).

4. Sonde (203) selon l'une quelconque des revendications précédentes, dans laquelle la distance axiale totale couverte par le premier ensemble de bobines et l'élément d'équilibrage est entre 19 mm et 30 mm.

5. Sonde (203) selon l'une quelconque des revendications précédentes, dans laquelle la ou chaque bobine d'entraînement a une longueur axiale entre 0,5 mm et 2,5 mm.

6. Sonde (203) selon l'une quelconque des revendications précédentes, dans laquelle la ou chaque bobine de détection a une longueur axiale entre 0,75 mm et 2 mm.

7. Sonde (203) selon l'une quelconque des revendications précédentes, dans laquelle la séparation axiale entre la première bobine et l'élément d'équilibrage est entre 12 mm et 15 mm.

8. Sonde (203) selon l'une quelconque des revendications précédentes, dans laquelle chaque bobine d'entraînement a un rayon moyen entre 1,5 mm et 6 mm.

9. Sonde (203) selon l'une quelconque des revendications précédentes, dans laquelle la ou chaque bobine de détection a un rayon extérieur entre 1,5 mm et 6,5 mm.

10. Sonde (203) selon l'une quelconque des revendications précédentes, dans laquelle la ou chaque bobine d'entraînement comprend entre 10 et 60 tours de fil.

11. Sonde (203) selon l'une quelconque des revendications précédentes, dans laquelle la ou chaque bobine de détection comprend entre 100 et 500 tours de fil.

12. Sonde (203) selon l'une quelconque des revendications précédentes, dans laquelle les bobines d'entraînement des premier et second ensembles de bobines sont connectées en série.

13. Sonde (203) selon l'une quelconque des revendications précédentes, dans laquelle la ou les bobines de détection du second ensemble de bobines sont couplées en anti-série avec la ou les bobines de détection du premier ensemble de bobines.

14. Sonde (203) selon l'une quelconque des revendications précédentes, dans laquelle la ou les bobines de détection du second ensemble de bobines sont enroulées dans une direction opposée à la ou aux bobines de détection du premier ensemble de bobines.

15. Sonde (203) selon l'une quelconque des revendications précédentes, dans laquelle le premier ensemble de bobines et l'élément d'équilibrage sont logés dans un boîtier de sonde, et dans laquelle le boîtier de sonde a un diamètre extérieur maximal entre 4 mm et 15 mm.

**16.** Système de détection pour localiser un marqueur magnétique, le système comprenant :

une sonde (203) à usage chirurgical selon l'une quelconque des revendications 1 à 15 ;
un générateur de champ magnétique agencé pour générer un champ magnétique alternatif à travers la ou les bobines d'entraînement du premier ensemble de bobines et l'élément d'équilibrage ; et
au moins un détecteur agencé pour recevoir un signal indiquant une tension de détection.

FIG. 1(a)
(For comparison)

FIG. 1(b)
(For comparison)

FIG. 2(a)
(For comparison)

FIG. 2(b)

(For comparison)

FIG. 3(a)

204a    204b

Side sense response

Position in axial direction

212

Side sense response

Position in axial direction

FIG. 3(b)

FIG. 3(c)

FIG. 4(a)

Distance in mm from probe housing tip

FIG. 4(b)

Probe former diameter (mm)

FIG. 4(c)

FIG. 4(d)

EP 4 505 199 B1

FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011067576 A1
- WO 2014140566 A1
- US 2012229130 A1
- WO 2016193753 A
- WO 2019180580 A
- WO 2014013235 A

**Non-patent literature cited in the description**

- A handheld SPIO-based sentinel lymph node mapping device using differential magnetometry. **WAANDERS S et al.** PHYSICS IN MEDICINE AND BIOLOGY. INSTITUTE OF PHYSICS PUBLISHING, 26 October 2016, vol. 61, 8120-8134